# EUROPEAN PATENT SPECIFICATION

(11) **EP 1 913 911 B1**
(45) Date of publication and mention of the grant of the patent: **01.02.2012**
(21) Application number: 07020198.3
(22) Date of filing: 16.10.2007
(51) Int. Cl.: A61F 13/02

(54) **An adhesive bandage, use of an adhesive bandage and a process for manufacturing and adhesive bandage**
Heftpflasterverband, Verwendung eines Heftpflasterverbands und Verfahren zur Herstellung eines Heftpflasterverbands
Bandage adhésif, utilisation d'un bandage adhésif et procédé de fabrication d'un bandage adhésif

(30) Priority: 16.10.2006 BR PI0604384
(43) Date of publication of application: 23.04.2008
(73) Proprietor: Johnson & Johnson Industrial Ltda., 12237-350 Sao José dos Campos-SP (BR)
(72) Inventor: Da Silva Macedo, Junior Carlos, Sao Jose dos Campos-SP (BR)
(74) Representative: Metten, Karl-Heinz

(56) References cited:
- EP-A- 0 413 251
- WO-A-03/057485
- WO-A-2004/112852
- CA-A1- 2 524 650
- FR-A- 2 736 833
- US-A- 4 622 089
- US-A- 5 681 579
- US-B1- 6 566 575

## Description

The present invention relates to an adhesive bandage to be applied specially to the skin, particularly an adhesive bandage that has great flexibility of use after having been applied to the skin, without, however, causing any damage to the injury upon removal and/or that has good absorption capacity for absorbing body exudates and provides good respirability. The invention further relates to a process for manufacturing this adhesive bandage, as well as to its use as a plaster or as a breast pad.

### Description of the prior art

Conventionally, adhesive bandages developed to be used on the skin, protecting it against injuries, or protecting the wounds from dirtiness, thus guaranteeing the efficacy of medicaments applied topically, comprise at least one film of a liquid-impermeable material, which prevents contamination of the wound as well.

At least one pad comprising at least one absorbent material and at least one adhesive element to attach the adhesive bandage to the skin is associated to the film. Preferably, the region intended for contact with the wound (the pad) does not have any adhesive, since the presence thereof might cause maceration in the injury upon removal and/or replacement of the adhesive bandage, which should be avoided so that the healing of the would can take place as rapidly as possible.

The acceleration of the healing is further enhanced by virtue of the fact that the film is permeable to gas and may optionally contain a plurality of through bores.

With a view to increase more and more the efficiency and comfort provided by a adhesive bandage, various improvements in this basic concept of adhesive bandage, which per se is extremely efficient, have been developed, as for example, improvement of the materials employed (more flexible, comfortable, cheap, with greater liquid-absorption capacity, etc.) or constitution of the bandage so as to prevent maceration / re-injury of the injury.

The first type of improved adhesive bandage is described in US Pat. 5,569,207 and is provided with at least one opening for positioning a catheter, needle or the like. This bandage is ultimately devised for use on patients who are using catheters or needles, and comprises essentially a layer of hydrocolloid material facing the wound, an intermediate layer of an absorbent foam for retaining body exudates and an outer film that is permeable to gases but impermeable to liquids and to penetration by bacteria.

A second type of hydrocolloid bandage is described in document EP 0 768 071 and has a thin layer of a mixture of hydrocolloid product and adhesive, which is provided on one side with a flexible and transparent backing layer and, on the other side, with one of more removable sheets, which enables one to secure it to the injured skin. This bandage further has a number of linear depressions that serve as guides for its positioning and possible bending, according to the needs to use it. In this type of bandage, the adhesive protecting sheets are not flexible, so that it can only be deformed after removal of these sheets, that is to say, only at the moment of using it.

A third type of improved adhesive bandage is described in US Pat. 5,250,043 and comprises essentially an adhesive layer, a pad of a super-absorbent element, a porous cover for this pad and a protective sheet. The pad cover comprises a plurality of orifices (openings) that enable body exudates to pass through and enhance the performance of the product.

A fourth type of hydrocolloid bandage is described in document EP 0 275 353 and comprises a perforated elastomeric film. This perforated polymeric film not only provides excellent properties of adhesion to the skin of the user, but also can be removed easily and with minimum re-injury.

A fifth type of improved adhesive bandage is described in US Pat. 5,486,158 and comprises a first layer constituted by a removable protective film, a second layer constituted by a high-absorption component including colloid and, finally, a third layer that is an impermeable cover sheet. A plurality of grooves is arranged circumferentially.

Adhesive bandages composed by a single liquid-permeable and gas-permeable film constitute another development of this type of product and are extremely thinner, comfortable and discreet, since they do not have any type of pad or similar absorbent element. In order for this type of bandage to absorb the body exudates, the adhesive layer contains in its composition at least one hydrocolloid compound, which absorbs emanated fluid to saturation.

The flexibility resulting from the thin thickness of the adhesive bandages composed by a single film cause them to be adhered to the skin for a long time, even when the injury is located in body-articulation regions, so that the replacement thereof is necessary after saturation of the absorbent capacity of the hydrocolloid. As a result, in addition to the discreetness and comfort, the number of adhesive bandages used until recovery of a skin injury is reduced, decreasing the expenditures of the user with the product.

The description of this type of adhesive bandage is great because, beyond reduced thickness, it is possible to configure the film in a way that it masks the existence of the bandage or makes it transparent, being just a little noted when in use.

In opposition to the advantages commented on above, the great disadvantage of the single-layer adhesive bandages is their high adhesion to the wound, which causes maceration /re-injury upon removal thereof for replacement after saturation. This is due to the impossibility of controlling the rate or intensity of adhesion of an adhesive bandage to the wound, since it is composed exclusively of this film having adhesive with hydrocolloid.

EP 0 413 251 A1 relates to bandages, in particular to bandages for applications requiring multiple bandages per day to the same area of the skin and which can be used for continent ostomates or others such as those suffering with mucous fistulas. These bandages comprise a flexible, thin, hydrocolloid adhesive layer having a plastic or non-woven fabric layer affixed to one surface thereof and a superabsorbent pad, smaller in area than the adhesive layer fixed to the opposite adhesive surface. The porous cover pad covers the superabsorbent pad and leaves a portion of the adhesive layer uncovered to form an adhesive border around the pads.

In WO 03/057485 A1 a multilayered wound dressing suitable for the management of especially chronic wounds is disclosed as well as a method for continuously manufacturing complex multilayered wound dressings. These wound dressings comprise laminates of several dissimilar materials in which the individual materials may be combined in various, predetermined configurations. Such method comprises a) providing a substantially continuous first web comprising a wound contact layer having a first and a second major phase and a first process release liner adhered to the first major face of the wound contact layer, b) forming a plurality of apertures through the wound contact layer, c) applying a discrete absorbent material layer from a substantially continuous web of the absorbent material layer to the second major phase of the wound contact layer, d) laminating a substantially continuous web of the backing layer over the absorbent material layer to form the composite web, and e) cutting individual multilayered wound dressings to form the composite web.

WO 2004/112852 A1 is about an antimicrobial composite comprising a first, liquid-permeable layer and a second layer arranged on said first layer, wherein an antimicrobial metal in elemental form is present between the first and second layers. Further, substantially no antimicrobial metal in element form has to be present on exterior surfaces of said composite.

CA 2,524,650 A1 describes an adhesive bandage consisting of at least one initial layer of structural film, at least one adhesive coating associated with the initial layer, and at least one pad. The initial layer of plastic film has to consist of one cavity for placement of the pad. Optionally, the adhesive bandage additionally consists of a second layer of a non-adhering, perforated plastic film placed over the pad as well as a covering layer. Such adhesive bandage shall easily adhere to the skin without the portions alongside the absorbent patent coming loose due to the areas of tension on its application surface such as the injured or wounded skin. Consequently, dirt is prevented from entering into the wound during use.

In US 5,681,579 a skin-contacting medical device is disclosed comprising a noncontinuous, hydrocolloid-containing skin facing polymeric support layer, an occlusive backing layer overlying said polymeric support layer, and an absorbent layer between and non-removably connected to said polymeric layer and said occlusive layer. US 5,681,579 also discloses a wound dressing comprising a) an occlusive layer of a polyurethane film, b) an absorbent region of a polymeric web, a fist layer of polypropylene fibers, a mixture of hydrocolloids and superabsorbents, and a second layer of polypropylene fibers, c) a polymeric support layer allowing for the rapid uptake of wound or body fluids, and d) a hydrocolloid-containing adhesive layer. These wound dressings shall exhibit superior absorption ability and shall be suited to absorb wound fluids at a greater rate for longer periods of time.

FR 2 736 833 A1 is about an adhesive bandage comprising a polymeric backing layer, an absorbent hydrocolloid-containing adhesive layer and a cover layer. Further embodiments comprise an additional non-adherent water permeable layer or a hydrocolloid adhesive layer wherein the hydrocolloid adhesive layer is substantially free of any absorbent material.

US 6,566,575 B1 relates to an absorbent dressing comprising a preselected patterned, hydrophilic gel absorbent layer comprising patterned elements having a width and a height, of independently, from 100 to 15.000 µm which can further comprise a permeable facing layer, a backing layer, and a pressure sensitive adhesive layer. It is reported that the absorbent layer can comprise a hydrocolloid dispersed in a hydrophobic polymer matrix. With such absorbent wound dressings it shall no longer be necessary to wash out the absorbent material from the wound.

Until the present moment an adhesive bandage had not been developed, particularly designed for being used as a dressing, which could join the best of conventional bandages provided with a pad (high absorption capacity, high respirability and good maceration/re-injury properties) with the advantages of single-film bandages, which are reduces thickness brining comfort and discreetness for the user.

### Objectives of the invention

An objective of the present invention is to provide an adhesive bandage for use on skin injuries, which has the characteristics of high peripheral-adhesion and absorption capability and, at the same time, provides comfort and discreetness for the user and has a good performance as far as maceration/re-injury of the wound, is concerned.

Also, the present invention has the objective of providing a process for manufacturing the aimed-at adhesive bandage.

### Brief description of the invention

The objectives of the present invention are achieved by means of an adhesive bandage according to the features of claim 1.

In a preferred embodiment the support layer comprises a polymeric film which is impermeable to liquids, but permeable to gases. The first composite layer comprises at least one first adhesive element and at least one first hydrocolloidal element and wherein the second composite layer comprises at least one second adhesive element and at least one second hydrocolloidal element. In another preferred embodiment the first composite layer is composed of at least one adhesive element and at least one first hydrocolloidal element, and/or the second composite layer is composed of at least one second adhesive element and at least one second hydrocolloidal element. It is possible but not necessary to employ identical first and second composite layers, or first and second composite layers at least the first and second adhesive elements or the first and second hydrocolloidal elements of which are essentially identical. In case the multi-layer system comprises one second composite layer and one permeable layer it is generally referred to as double layer system. According to one embodiment the permeable layer of said double or multi-layer system comprises a perforated layer, for example perforated film.

According to the present invention an adhesive bandage is provided wherein at least one segment of the double or multi-layer system is associated to the first composite layer via its second composite layer. In this regard, the second composite layer preferably is associated to a section of the first composite layer and the permeable layer is associated to the second composite layer.

According to the present invention an adhesive bandage is provided wherein at least one segment of the double or multi-layer system is associated to the first composite layer via its permeable layer. In this regard, the permeable layer preferably is associated to a section of the first composite layer and the second composite layer is associated to the permeable layer.

According to a preferred embodiment of the present invention an adhesive bandage is provided wherein at least a first segment of the double or multi-layer system is associated to the first composite layer via its second composite layer and wherein at least one second segment of the double or multi-layer system is associated to the first composite layer via its permeable layer.

According to another preferred embodiment the adhesive bandage according to the present invention is characterized in that the segment of the double or multi-layered system being associated to the first composite layer and having a second surface area is smaller than the first surface area.

Suitable hydrocollodial elements of the first and/or second composite layer comprise carboxymethyl cellulose, pectin, xanthan gum, polysaccarides, alginates, chitosan, marine algae extract, polyaspartic acid, polyglutamic acid, hyaluronic acid or salts and derivatives as well as mixtures thereof. Suitable adhesive elements of the first and/or second composite layer comprise, for example, a pressure acrylic adhesive.

Furthermore, suitable support layer materials comprise polyolefins, polyurethanes, polyethylene vinyl acetate, non-wovens, rubber materials, textiles or mixtures thereof.

Adhesive bandages according to the present invention also include those wherein at least one permeable layer and at least one second composite layer comprises a plurality of through bores, in particular through bores which are essentially perpendicular to the first surface (S1) of the support layer. With the adhesive bandages of the present invention at least one through bore goes beyond said permeable layer and said second composite layer and reaches through at least part of the composite layer. It is particularly preferred that at least one, in particular a plurality of through bores of the permeable layer and the second composite layer are located in a matching position so that a contiguous passageway is generated. According to another preferred embodiment at least one through bore, in particular a plurality of through bores which reaches/reach through at least part of the composite layer is/are located in matching position with the through bore(s) of the permeable layer and the through bore(s) of the second composite layer thereby generating a contiguous passageway. These passageways are preferably essentially perpendicular to the first surface (S1).

According to another aspect of the present invention the underlying object has been solved by a process for the manufacture of an adhesive bandage according to the features of claim 6.

A preferred process further comprises the step of shaping the double or multi-layer system obtained in step (iv), so that it covers an area smaller than the area covered by the support layer.

In a preferred embodiment the steps (i) and (iii) and/or (ii) and (iv) take place either concomitantly or at separate moments. The process of the present invention comprises providing at least one through bore, in particular a plurality of through bores, through the permeable layer and/or the second composite layer.

### Brief description of the drawings

The present invention will now be described in greater detail with reference to an embodiment represented in the drawings. The figures show.
- Figure 1 is a schematic perspective view of a first embodiment of the adhesive bandage of the present invention;
- Figure 2 corresponds to a schematic sectional view of the adhesive bandage illustrated in figure 1;
- Figure 3 is a schematic perspective view of the bandage illustrated in figure 1 with the thickness of the central portion quite highlighted;
- Figure 4 corresponds to a schematic sectional view of the bandage illustrated in figure 3, and where its thickness is quite highlighted;
- Figure 5 corresponds to a schematic sectional view of a second embodiment of the bandage illustrated in figure 3, and where its thickness is quite highlighted;
- Figure 6 is a schematic perspective view of a conventional adhesive bandage with an absorbent pad; and
- Figure 7 corresponds to a schematic sectional view of the conventional adhesive bandage illustrated in figure 6.

### Detailed description of the figures

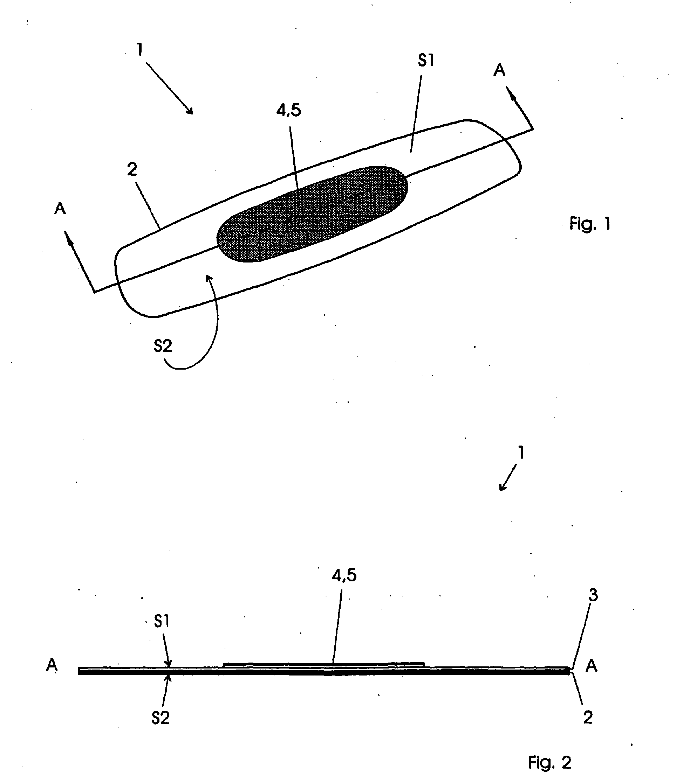

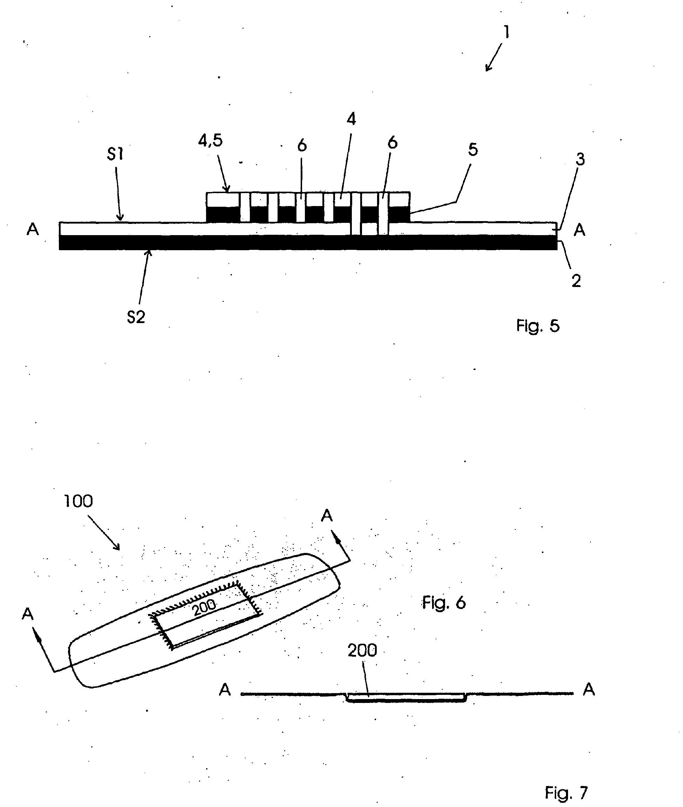

Figures 1 to 5 illustrate two possible embodiments of the adhesive bandage 1 of the present invention, while figures 6 and 7 illustrate a conventional prior-art bandage 100 provided with an absorbent pad 200.

The conventional bandage 100 illustrated in figures 6 and 7 comprises a main impermeable plastic film, to which quite thick absorbent pad 202 is associated, preferably by means of glue. The region dominated by the pad 200 does not contain any kind of adhesive, since it should not at all adhere to the wound, at the risk of causing additional injuries when the adhesive bandage 100 is removed from the skin (re-injury).

This type of conventional adhesive bandage 100, although efficient with regard to the capability of absorbing body exudates from the healing wound, due to the fact that the absorbent pad 200 is quite thick, exhibit little flexibility and ends up detaching from the skin of the user long before saturation, thus requiring an early replacement. Moreover, this type of bandage is not efficient in use as a skin protector or as breast protector or as a sanitary napkin.

Another type of bandage used is the one made from single-layer film without any absorbent pad, which is not illustrated in the figures because of its simplicity. By virtue of the characteristics of the component materials and to its reduced thickness, the single-layer adhesive bandage is highly flexible and so it can remain adhered to the skin for a long time, even if positioned in body-articulation regions. With a view to guarantee the absorption of body fluids, at least one hydrocolloid element mixed with the adhesive is applied.

By virtue of its high flexibility, this type of bandage remains in use without detaching until the hydrocolloid becomes saturated, and then it is replaced. Optionally, this type of adhesive bandage may also be configured so as to mask, as much as possible, its existence (presenting a finish of the sake color as the skin, frosted translucent, etc.).

The great disadvantage of this type of bandage lies in that fact that the mixture of adhesive and hydrocolloid is applied throughout its extent, and this is necessary above all in the region that covers the wound, since it is there that the presence of the hydrocolloid with its absorption capacity is crucial. However, since the hydrocolloid is associated with the adhesive, the wound region may undergo re-injury upon removal of the bandage.

The first configuration of the adhesive bandage 1 of the present invention, illustrated in figures 1 to 4, in turn, does not have this drawback. It comprises at least one polymeric film 2, called also support layer, to which at least one first layer 3 composed of at least one adhesive element and at least one hydrocolloid element is associated.

The first support layer 2 may be of various shapes, as required (rectangular, circular, oblong, etc.), and its shape will that presented by the adhesive bandage 1, that is to say, the shape of the adhesive bandage is defined by the first support layer 2. Likewise, the composition of the first support layer 2 may vary, but it is preferably manufactured from a polyolefin film, polyurethane polymer, polyethylene, polyethylene vinyl acetate, polyurethane foam, and may still be made from a textile, non-woven material, rubber, etc.

The polymeric film or support layer 2 has a first surface S1 facing the skin of the user and a second opposite surface S2. The first surface S1 defines or has an area value called first surface area. The first layer (3) composed of adhesive and hydrocolloid is applied over the whole surface area defined by the first surface S1 (therefore, obviously facing the skin of the user).

Further preferably, the first support layer 2 is impermeable to liquids, but permeable to gases, which allows the injury and the skin to which the adhesive bandage 1 is adhered to breathe. In order for this to be possible, the support layer or the polymer comprises pores sized so as to allow only gases to pass, which are composed of molecules known to be small. Finally, one may conceive a layer 2 that is perforated in the regions that will not come in contact with the injury, further increasing skin ventilation. Alternatively, the first support layer 2 may further be totally impermeable to gases, when necessary.

The first composite layer 3, in turn, comprises at least one discreet hydrocolloid element. By "discreet elements" one understands particles of reduced size dispersed in the adhesive, as taught in patents US 5,643,187 and US 6,558,792, incorporated herein by reference. The hydrocolloid element used may be any substance that has a good performance in this use, as for example, sodium carboxymethyl cellulose, pectin, xanthan gum, polysaccharides, sodium or calcium alginates, chitosan, marine algae extract / (carrageenan), polyaspartic acid, polyglutamic acid, hyaluronic acid or salts and derivatives thereof, among others.

Hydrocolloids, such as sodium carboxymethyl cellulose and pectin, among others, are agents that form gels as soon as they come into contact with the body fluids emanated from injuries. In the use of adhesive bandages, these hydrocolloids are combined with elastomers and/or adhesives (the composition of which will be mentioned later). Preferably, the adhesive bandage 1 should guarantee a moistened environment, but without saturation, for healing, a situation favorable to the acceleration thereof.

Pectin is a complex-structure polysaccharide extracted from plant species (such as citric-fruit barks and apple pulp), which has a highly hydrophilic structure and, consequently, associates easily with the water molecules of body fluids emanated through the wound, forming a viscous gel in the injury bed. Its chemical similarity with alginates causes the physical properties of absorption and formation of gel to resemble each other.

Caraboxymethyl cellulose, in turn, is a cellulose derivative formed by reaction of cellulose with alkalis (such as sodium, potassium, calcium hydroxide, etc) and chloroacetic acid. It is the nature of the combined alkali that differentiates the ionic characteristic of carboxymethyl cellulose (when using sodium hydroxide, sodium carboxymethyl cellulose is formed). Like what occurs with pectin, carboxymethyl cellulose dissolves rapidly in the water of the fluids emanated through the wound, forming a gel in the wound with controlled viscosity.

As an additional advantage of the use of hydrocolloids, one should note that both pectin and carboxymethyl cellulose form a gel of acidic characteristics (pH of about 4), functioning as an antibacterial agent.

Prior to use of the adhesive bandage 1, hydrocolloid is substantially inert to water vapor, but as soon the gelling process begins, the adhesive bandage 1 becomes progressively more permeable. The gelling process continues, as long as the wound continues to release body fluids, until the hydrocolloid is used, and then the adhesive bandage 1 reaches saturation and should be replaced.

In the same way, the adhesive element used may be any one, as for example pressure acrylic adhesive, among others. Additionally, such an adhesive may contain a resin for increasing adhesion, a cohesion increasing agent, an absorbing agent (preferably a polyacrylate super-absorbent, a polyacrylate-salt super-absorbent or mixture thereof), a plasticizer and optionally a pigment. The first composite layer 3 may further be configured in discontinuous patterns, exhibiting an arrangement in lines, web, spray or any other that a parson skilled in the art considers to be continuous.

At least one segment of perforated polymeric film 4,5 is associated to the first layer 3, which has a second surface substantially smaller than the first one (the surface area defined by the first surface S 1 of the support layer 2).

The segment of perforated polymeric film 4,5 is preferably but not compulsorily positioned centralized with respect to the support layer 2 and, as will be discussed in detail later, it has the function of acting over the injury on the skin of the user, preventing re-injury and keeping good properties as far as maceration is concerned.

In order to perform this function, the segment of perforated polymeric film 4,5 comprises at least one layer of permeable polymeric film 5, to which one associates at least one second composite layer 4 formed by at least one adhesive element and at least one hydro colloidal element.

By preference, the permeable layer 5 is similar in constitution to the support layer 2 and the second composite layer 4 is similar to the first composite layer 3, for the sake of manufacturing ease, but it is evident that both of them may have any necessary or desirable constitutions, provided that they are functional.

In this first configuration of the bandage illustrated in figures 1 to 4, the association of the segment of perforated polymeric film 4,5 to the support layer 2 is effected by associating the second composite layer 4 to the first composite layer 3. Therefore, it is the permeable layer 5 that remains facing the skin of the user when the bandage is ready to be used, and this is advantageous, since no adhesive is applied to the user-facing surface of this layer 5.

The second configuration of the bandage of the present invention, illustrated in figure 5, is identical to the first one in almost all aspect, with the exception of the fact that the segment of polymeric film is mounted inverted, that is to say, the permeable layer 5 of the segment 4, 5 is associated to the first composite layer 3 and the second composite layer 4 faces the skin of the user. This constitution brings some differences to the performance of the bandage, which make it suitable for some uses and will be described later.

With a view to protect the bandage 1 until it is applied, one preferably provides a sheet of non-stick material (not shown) having a shape analogous to that of the support layer or the like, which remains positioned in contact with the first composite layer 3 and (i) with the permeable layer 5 (with the latter in the region where the segment of perforated film is associated to the support layer 2) in the case of the first embodiment of the bandage or (ii) with the second composite layer 4 (also in the region where the segment of perforated film is associated to the support layer 2) in the case of the second embodiment of the bandage.

Thus, one preserves (i) the adhesion capability of the first composite layer 3, (ii) the hydrocolloidal properties, and (iii) the hygiene of the permeable layer 5 or of the second composite layer 4 (depending on the configuration of the bandage), which will be in contact with the injury.

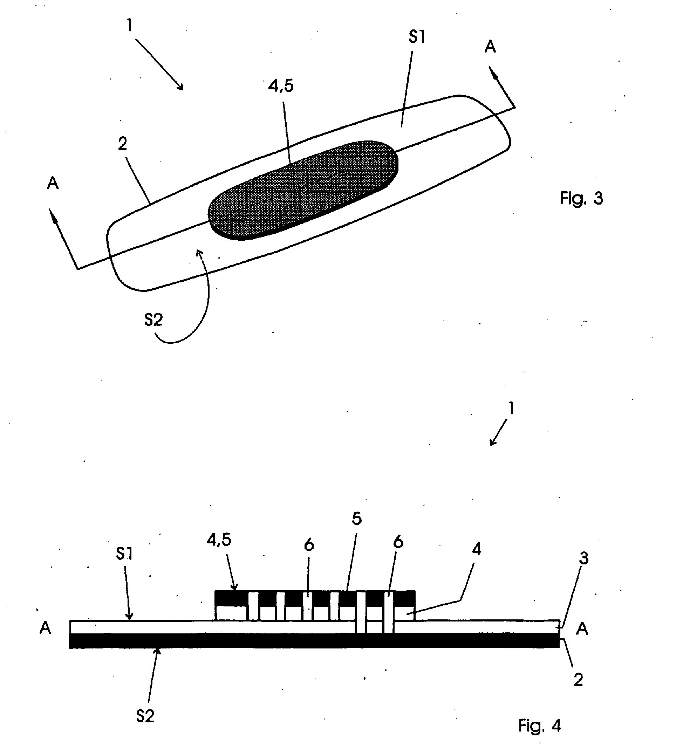

The segment of permeable polymeric film 4,5 comprises a plurality of substantially perpendicular through bores 6 that passes through both the polymeric film 5 (making it permeable) and the second composite layer 4. At least one of the through bores 6 goes beyond the polymeric film layer 5 and the second composite layer 4, and even reach the first composite layer 3. In the two situations mentioned the bores 6 can be seen in schematic enlargements in figures 4 and 5, since the diameter is very reduced and the their length is even smaller.

The through bores 6 correspond to real channels for carrying body fluids, enabling them to be conducted by capillarity away from the discharge region (the skin injury) and to be absorbed by the hydro colloidal elements. For this reason the segment of permeable polymeric film 4, 5 comprises the second composite layer 4, which increases the amount of hydrocolloid and, by inference, the absorption capacity of the bandage 1.

For use of the first embodiment of the bandage 1, the user, after removing the sheet of non-stick material, should position it in such a way that the polymeric film layer 5 will be positioned over the injury, preventing the first composite layer 3 from being thus positioned. Since there is no adhesive on the surface of the polymeric film layer 5 facing the user, there is no risk that the portion will stick to the injury, causing re-injury upon removal of the bandage 1.

After positioning the bandage 1, the body liquids emanated from the injury pass through the bores 6 and through the polymeric film layer 5 (which, with the exception of the bores 6, is impermeable), reaching the second composite layer 4. At this moment, the liquids begin to react with the hydrocolloid, remaining stored. The bores 6 further conduct the liquids as far as the first composite layer, which is larger in area, where the large amount of hydro colloidal material changes into a great absorption capacity.

Since there is no adhesive on the surface of the polymeric film layer 5 facing the user, there is no risk that this portion sticks to the wound, causing re-injury upon removal of the bandage 1.

On the other hand, for use of the second embodiment of the bandage 1, the user removes the sheet of non-stick material and positions the bandage in such a way that the second composite layer 4 will be positioned over the injury. In this second embodiment, since the second composite layer 4 comprises a mixture of adhesive and hydrocolloid, the risk of re-injury is not so low, but on the other hand the absorption capacity is maximized, since the hydrocolloid remains in direct contact with the injury.

The second embodiment of the bandage is more suitable, for instance, for use in dressings where one desires higher velocities and absorption capacity, a situation where the saturation of the bandage occurs more rapidly. In this regard, one should further mention that, upon saturation of the hydrocolloid present in the second composite layer 4, there is natural displacement thereof, which attenuates the possibility of re-injury. In this second embodiment, the bores 6 act as described before.

It is important to note that the segment of permeable polymeric film 4,5 positioned over the first composite layer 3 brings an extremely reduced increase in the thickness of the bandage 1, since its thickness is the same (or almost the same) as that of the bandage prior to application. Besides, this extremely reduced increase in the thickness will occur only in the region where this segment is applied. The bandage 1 then has a thickness as reduced as that of bandages made from a single-layer film of the prior art, with the great advantage of reducing to virtually zero the chances of re-injury / maceration.

Thus, with the present bandage 1, it is possible to obtain the advantages of the reduced thickness of the monolayers bandages together with the capability to prevent re-injury of bandages with pads, and a capability of sufficient absorption due to the existence of the hydrocolloid element in the first and second composed layers 3,4.

In addition to the above example, the adhesive bandage 1 of the present invention can be used, for instance, as a breast protection. To this end, it is enough to handle adequately the variables such as areas of the support layer 2 and of the segment of permeable polymeric film 4,5.

It is further possible for the bandage to be one of the components of a breast protector or an absorbent composed of further elements and layers, so that the absorption desired will be achieved.

A process for manufacturing the adhesive bandage of the present invention can comprise the following steps:
(i) preparing and cutting the polymeric film 2;
(ii) applying at least one first composite layer 3 to a first surface S 1 of the polymeric film 2;
(iii) preparing and cutting a layer of polymeric film 5 ;
(iv) applying at least one second composite layer 4 to the layer of perforated polymeric film 5, shaping the perforated polymeric segment 4,5;
(v) associating the perforated polymeric segment 4,5 to the first composite layer 3 of the polymeric film 2; and
(vi) making a plurality of through bores 6.

Evidently, the steps (i) and (iii) of preparing and cutting the polymeric layer 2 and of the layer of polymeric film 5, respectively, may occur either concomitantly or at separate moments, since this makes no difference to the effect of protecting the invention. In the same way, this may happen with the steps (ii) and (iv).

Preferably, the step (vi) of making the through bores 6 is carried out at the end, but nothing prevents one from making these bores before, as for instance at the time of applying the first and the second composite layers 3,4 (steps ii and iv), or still at another time.

A preferred embodiment having been described, one should understand that the scope of the present invention embraces other possible variations, being limited only by the contents of the accompanying claims, which include the possible equivalents.

## Claims

1. An adhesive bandage (1) comprising a support layer (2), which has a first surface (S1) facing the skin of the user and a second opposite surface (S2), the first surface (S1) defining a first surface that receives at least partially an application of at least one first composite layer (3), the bandage (1) being **characterized in that** at least one segment of at least one double or multi-layer system (4, 5) is associated to at least a section of the composite layer (3) wherein the double or multi-layer system (4, 5) comprises at least one permeable layer (5) and at least one second composite layer (4); the first composite layer (3) comprises art least one first adhesive element and at least one first hydrocolloidal element and wherein the second composite layer (4) comprises at least one second adhesive element and at least one second hydrocolloidal element; wherein at least one permeable layer (5) and at least one second composite layer (4) comprises a plurality of through bores (6); wherein the support layer (2) is impermeable to liquids; and wherein the at least one segment of the double or multi-layer system (4, 5) is associated to the first composite layer (3) via its second composite layer (4) or wherein the at least one segment of the double or multi-layer system (4, 5) is associated to the first composite layer (3) via its permeable layer (5), and wherein at least one through bore (6) goes beyond said permeable layer (5) and said second composite layer (4) and reaches through at least part of the composite layer (3).

2. The adhesive bandage (1) according to claim 1, wherein the segment of the double or multi-layered system (4, 5) being associated to the first composite layer (3) and having a second surface area is smaller than the first surface area.

3. The adhesive bandage(1) according to claim 1 or 2, further comprising at least one sheet of non-stick material having a shape analogeous to that of the support layer (2) and which is releasably associated to that side of the bandage applied to a surface which is opposite the second surface (S2).

4. The adhesive bandage(1) according to any of the preceding claims, wherein
such a bandage represents a plaster or a breast pad.

5. Use of an adhesive bandage (1) according to any of the preceding claims as a plaster or as a breast pad.

6. Process for the manufacture of an adhesive bandage(1) according to claims 1 to 4, comprising the following steps:
(i) providing a support layer (2), which is impermeable to liquids, having a first surface (S1) and an opposite second surface (S2),
(ii) applying at least one first composite layer (3) to at least part of the first surface (S1) of said support layer (2),
(iii) providing at least one permeable layer (5),
(iv) applying at least one second composite layer (4) to the permeable layer (5), to form a double or multi-layer system (4, 5), and
(v) associating at least a section of the double or multi-layer system (4, 5) to the first composite layer (3) via the permeable layer (5) or associating at least a section of the double or multi-layer system (4, 5) to the first composite layer (3) via the second composite layer (4),
wherein said first and said second composite layers (3, 4) comprise adhesive and hydrocolloid. and wherein said permeable layer (5) and said second composite layer (4) comprise a plurality of through bores (6), and wherein at least one through bore (6) is provided which reaches through at least part of the composite layer (3).

7. The process according to claim 6, further comprising the step of shaping the double or multi-layered system (4, 5) obtained in step (iv) so that it covers an area smaller than the area covered by the support layer (2).

8. The process according to claim 6 or 7, wherein the steps (i) and (iii) and/or (ii) and (iv) take place either concomitantly or at separate moments.

## Patentansprüche

1. Haftende Binde (1), umfassend: eine Tragschicht (2), die eine erste Oberfläche (S1), die zur Haut des Benutzers weist, und eine zweite gegenüberliegende Oberfläche (S2) aufweist, wobei die erste Oberfläche (S1) einen ersten Flächeninhalt definiert, der mindestens teilweise eine Aufbringung mindestens einer ersten Verbundstoffschicht (3) erhält, wobei die Binde (1) **dadurch gekennzeichnet ist, dass** mindestens ein Segment mindestens eines Zwei- oder Mehrschictsystems (4, 5) mit mindestens einem Abschnitt der Verbundstoffschicht (3) verbunden ist, wobei das Zwei-oder Mehrschichtsystem (4, 5) mindestens eine durchlässige Schicht (5) und mindestens eine zweite Verbundstoffschicht (4) umfasst; wobei die erste Verbundstoffschicht (3) mindestens ein erstes haftendes Flement und mindestens ein erstes hydrokolloidales Flement umfasst und wobei die zweite Verhundstoffschicht (4) mindestens ein zweites haftendes Element und mindestens ein zweites hydrokolloidales Element umfasst; wobei mindestens eine durchlässige Schicht (5) und mindestens eine zweite Verbundstoffschicht (4) eine Mehrzahl durchgehender Bohrungen (6) umfasst; wobei die Tragschicht (2) flüssigkeitsundurchlässig ist; und wobei das mindestens eine Segment des Zwei- oder Mehrsehichtsystems (4. 5) über seine zweite Verbundstoffschicht (4) mit der ersten Verbundstoffschicht (3) verbunden ist oder wobei das mindestens eine Segment des Zwei- oder Mehrschichtsystems (4, 5) über seine durchlässige Schicht (5) mit der ersten Verbundstoffschicht (3) verbunden ist und wobei mindestens eine durchgehende Bohrung (6) über die durchlässige Schicht (5) und die zweite Verbundsloffschicht (4) hinaus geht und durch mindestens einen Teil der Verbundstoffschicht (3) reicht.

2. Haltende Binde (1) nach Anspruch 1, wobei das Segment des Zwei-oder Mehrschichtsystems (4, 5), das mit der ersten Verbundstoffschicht (3) verbunden ist und einen zweiten Flächeninbalt aufweist, kleiner ist als der erste Flächeninhalt.

3. Haftende Binde (1) nach Anspruch 1 oder 2, ferner unfassend; mindestens ein flächiges Element aus nichthaftendern Material. das eine Form analog zu derjenigen der Tragschicht (2) aufweist und das lösbar mit der Seite der Binde verbunden ist, die auf eine Oberfläche aufgebracht ist, die der zweiten Oberfläche (S2) gegenüberliegt.

4. Haltende Binde (1) nach einem der vorangehenden Ansprüche, wobei eine solche Binde ein Master oder ein Brustpad darstellt.

5. Verwendung einer haftenden Bindd (1) nach einem der vorangehenden Ansprüche als Pflaster oder Brustpad.

6. Verfahren zur Herstellung einer haftenden Binde (1) nach den Ansprüchen 1 bis 4, das folgende Schritte umfasst:
(i) Bereitstellen einer Tragschicht (2), die flüssigkeistsundurchlässig ist, die eine erste Oberfläche (S1) und eine gegenüberliegende zweite Oberfläche (S2) aufweist,
(ii) Aufbringen mindeslens einer ersten Verbundstoffschicht (3) auf mindestens cinen Teil der ersten Oberfläche (S1) der Tragschicht (2),
(iii) Bereitstellen mindestens einer durchlässigen Schicht (5),
(iv) Aufbringen mindestens einer zweiten Verbundstoffschicht (4) auf die durchlässige Schicht (5), um ein Zwei- oder Mehrschichtsystem (4, 5) auszubilden, und
(v) Verbinden mindestens einer Abschnitts des Zwei- oder Mchrschichtsystem (4, 5) mit der ersten Verbundstoffschicht (3) über die durchlässige Schicht (5) oder Verbinden mindestens eines Abschnitts des Zwei- oder Mehrschichtsystems (4, 5) mit der ersten Verbundstoffschicht (3) über die zweite Verbundstoffschicht (4),
wobei die erste und die zweite Verbundsloffschicht (3, 4) Haftmittel und Hydrokolloid umfassen und wobei die durchlässige Schicht (5) und die zweite Verbundstoffschicht (4) eine Mehrzahl durchgehender Bohrungen (6) umfassen und wobei mindestens eine durchgehende Bohrung (6) vorgesehen ist, die durch mindestens einen Teil der Verbundstoffschicht (3) reicht.

7. Verfahren nach Anspruch 6, ferner umfassend; den Schritt des Formens des in Schritt (iv) erhaltenen Zwei- oder Mehrschichtsystems (4, 5), so dass es eine Fläche bedeckt, die kleiner als die von der Tragschicht (2) bedeckte Fläche ist.

8. Verfahren nach Anspruch 6 oder 7, wobei die Schritte (i) und (iii) und/oder (ii) und (iv) entweder gleichzeitig oder zu getrennten Zeitpunkten erfolgen.

## Revendications

1. Bande (1) adhésive comprenant une couche support (2), qui a une première surface (S1) faisant face à la peau de l'utilisateur et une deuxième surface (S2) opposée, la première surface (S1) définissant une première aire de surface qui reçoit au moins partiellement une application d'au moins une première couche (3) composite, la bande (1) étant **caractérisée en ce qu'**au moins un segment d'au moins un système (4, 5) double ou multicouche est associé à au moins une section de la couche (3) composite dans laquelle le système (4, 5) double ou multicouche comprend au moins une couche (5) perméable et au moins une deuxième couche (4) composite ; la première couche (3) composite comprend au moins un premier élément adhésif et au moins un premier élément hydrocolloïdal, et dans laquelle la deuxième couche (4) composite comprend au moins un deuxième élément adhésif et au moins un deuxième élément hydrocolloïdal ; dans laquelle au moins une couche (5) perméable et au moins une deuxième couche (4) composite comprennent une pluralité de trous débouchants (6) ; dans laquelle la couche support (2) est imperméable aux liquides ; et dans laquelle le segment, au moins au nombre de un, du système (4, 5) double ou multicouche est associé à la première couche (3) composite via sa deuxième couche (4) composite, ou dans laquelle le segment, au moins au nombre de un, du système (4, 5) double ou multicouche est associé à la première couche (3) composite via sa couche (5) perméable, et dans laquelle au moins un trou débouchant (6) va au-delà de ladite couche (5) perméable et de ladite deuxième couche (4) composite et traverse au moins une partie de la couche (3) composite.

2. Bande (1) adhésive selon la revendication 1, dans laquelle le segment du système (4, 5) double ou multicouche qui est associé à la première couche (3) composite et qui a une deuxième aire de surface est plus petit que la première aire de surface.

3. Bande (1) adhésive selon la revendication 1 ou 2, comprenant également une feuille de matériau anti-adhérant ayant une forme analogue à celle de la couche support (2) et qui est associée de façon détachable au côté de la bande qui est appliqué sur une surface qui est opposée à la deuxième surface (S2).

4. Bande (1) adhésive selon une quelconque des revendications précédentes, dans laquelle une telle bande représente un emplâtre ou un coussinet pour sein.

5. Utilisation d'une bande (1) adhésive selon une quelconque des revendications précédentes en tant qu'emplâtre ou en tant que coussinet pour sein.

6. Procédé de fabrication d'une bande (1) adhésive selon les revendications 1 à 4, comprenant les étapes suivantes :
(i) la fourniture d'une couche support (2) qui est imperméable aux liquides, ayant une première surface (S1) et une deuxième surface (S,2) opposée,
(ii) l'application d'au moins une première couche (3) composite sur au moins une partie de la première surface (S1) de ladite couche support (2),
(iii) la fourniture d'au moins une couche (5) perméable,
(iv) l'application d'au moins une deuxième couche (4) composite sur la couche (5) perméable pour former un système (4, 5) double ou multicouche, et
(v) l'association d'au moins une section du système (4, 5) double ou multicouche à la première couche (3) composite via la couche (5) perméable, ou l'association d'au moins une section du système (4, 5) double ou multicouche à la première couche (3) composite via la deuxième couche (4) composite,
dans lequel lesdites première et deuxième couches composites (3, 4) comprennent adhésif et hydrocolloïde, et dans lequel ladite couche (5) perméable et ladite deuxième couche (4) composite comprennent une pluralité de trous débouchants (6), et dans lequel au moins un trou débouchant (6) est prévu, qui traverse au moins une partie de la couche (3) composite.

7. Procédé selon la revendication 6, comprenant également l'étape consistant à donner une forme au système (4, 5) double ou multicouche obtenu dans l'étape (iv) de sorte qu'il couvre une aire plus petite que l'aire couverte par la couche support (2).

8. Procédé selon la revendication ou 7, dans lequel les étapes (i) et (iii) et/ou (ii) et (iv) sont effectuées soit de façon concomitante, soit à des moments séparés.
